(19) Europäisches Patentamt
European Patent Office
Office européen des brevets

(11) EP 1 086 073 B1

(12) **FASCICULE DE BREVET EUROPEEN**

(45) Date de publication et mention de la délivrance du brevet:
**12.03.2003 Bulletin 2003/11**

(21) Numéro de dépôt: **00914251.4**

(22) Date de dépôt: **24.03.2000**

(51) Int Cl.[7]: **C07C 239/20**

(86) Numéro de dépôt international:
**PCT/FR00/00750**

(87) Numéro de publication internationale:
**WO 00/061544 (19.10.2000 Gazette 2000/42)**

(54) **PROCEDE DE PREPARATION D'ALCOXYAMINES A PARTIR DE NITROXYDES**

VERFAHREN ZUR HERSTELLUNG VON ALKOXYAMINS AUS NITROXIDEN

METHOD FOR PREPARING ALKOXYAMINES FROM NITROXIDES

(84) Etats contractants désignés:
**AT BE CH CY DE DK ES FI FR GB GR IE IT LI LU MC NL PT SE**

(30) Priorité: **08.04.1999 FR 9904405**

(43) Date de publication de la demande:
**28.03.2001 Bulletin 2001/13**

(73) Titulaire: **Elf Atochem S.A.**
**92800 Puteaux (FR)**

(72) Inventeurs:
- **COUTURIER, Jean-Luc**
  **F-69006 Lyon (FR)**
- **GUERRET, Olivier**
  **F-69280 Marcy l'Etoile (FR)**
- **SENNINGER, Thierry**
  **F-57700 Hayange (FR)**

(56) Documents cités:
**EP-A- 0 157 738** **WO-A-98/40415**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 086 073 B1

Printed by Jouve, 75001 PARIS (FR)

## Description

La présente invention a pour objet un procédé de préparation d'hydroxylamines α,β,β-trisubstituées, ci-après désignées par alcoxyamines, obtenues à partir de nitroxydes, utilisables notamment comme amorceurs des polymérisations radicalaires. L'utilisation des alcoxyamines telles que celles dérivées du (2,2,6,6-tétraméthylpipéridiriyl)-N-oxyde (TEMPO) dans la préparation des macromolécules a donné lieu à de nombreuses publications.

Ainsi, Hawker C.J. et coll. (Macromolécules 1996, 29, pages 5245-5254) ont montré que l'utilisation d'alcoxyamines dérivées du TEMPO telles que le (2',2',6',6'-tétraméthyl-1'-pipéridinyloxy)méthylbenzène comme amorceurs de polymérisation radicalaire du styrène permettait de contrôler la polymérisation et d'accéder à des polymères bien définis avec de bas indices de polydispersité et ils ont constaté que les vitesses de polymérisation étaient sensiblement équivalentes aux vitesses obtenues lorsqu'ils utilisaient des amorceurs classiques tels que l'AIBN ou le peroxyde de benzoyle en présence de TEMPO.

Les alcoxyamines peuvent être préparées selon des méthodes connues dans la littérature. La méthode la plus courante implique le couplage d'un radical carboné avec un radical nitroxyde.

Si on désigne par :

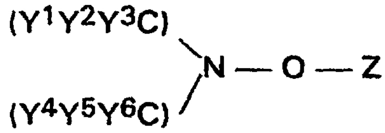

(I)

une alcoxyamine, $Y^1$, $Y^2$, $Y^3$, $Y^4$, $Y^5$, $Y^6$, Z étant définis plus loin, le radical carboné $Z^{\bullet}$ peut être généré par différentes méthodes décrites dans la littérature : décomposition d'un composé azoïque, abstraction d'un atome d'hydrogène sur un substrat approprié, addition d'un radical sur une oléfine. Le radical $Z^{\bullet}$ peut également être généré à partir d'un composé organométallique comme un organomagnésien Z-MgX tel que décrit par Hawker C.J. et coll. dans Macromolécules 1996, 29, 5245-5254 ou à partir d'un dérivé halogéné Z-X en présence d'un système organométallique comme CuX/bipyridine (X=Cl ou Br) selon une réaction de type ATRA (Atom Transfer Radical Addition) tel que décrit par Dorota Greszta et coll. dans Macromolécules 1996, 29, 7661-7670.

Une des méthodes les plus utilisées pour la préparation des alcoxyamines (I) est la méthode mettant en jeu la réaction ATRA.

Cette méthode consiste à transférer un atome ou un groupe d'atomes sur une autre molécule en présence d'un système organométallique CuX/bipyridine, en milieu solvant selon le schéma ;

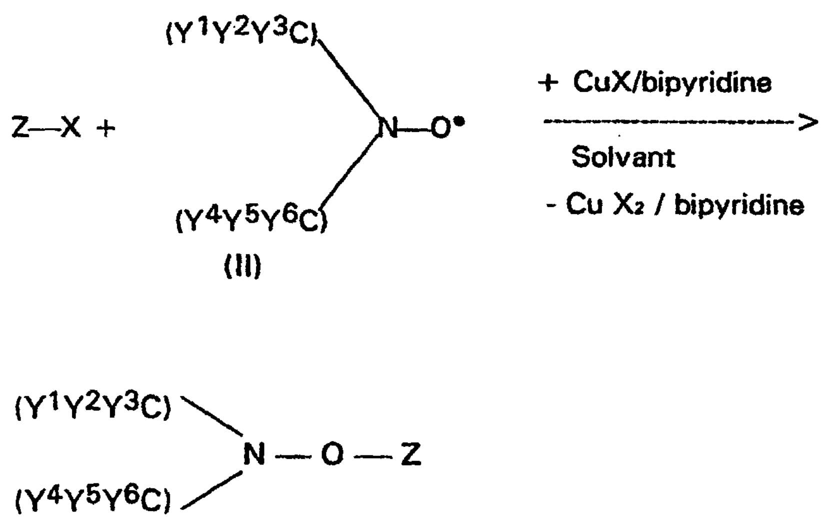


(II)

(I)

De préférence, X dans le système organométallique représente un atome de brome.

Le mode opératoire généralement utilisé consiste à mettre en solution le système organométallique tel que

CuBr/bipyridine dans un solvant organique de préférence aromatique tel que le benzène ou le toluène, puis à introduire dans la solution le composé ZX et le nitroxyde (II).

Cette façon d'opérer présente l'inconvénient majeur de nécessiter des durées de réactions longues, rédhibitoires pour une préparation industrielle d'alcoxyamines ou d'utiliser un large excès d'un des réactifs.

De plus, le système organométallique utilisé met en jeu des ligands coûteux (bipyridine ou dérivés).

En outre, l'élimination du métal résiduel des produits obtenus est difficile,. nécessitant des opérations coûteuses de purification telles que le passage des produits sur colonne de silice.

Ainsi, par exemple Matyjaszewski K. et coll. dans la demande de brevet internationale WO 98/40415 obtiennent le 1-(2,2,6,6-tétraméthylpipéridinyloxy)-1-phényléthane avec un rendement de 69 % après purification par chromatographie sur colonne en faisant réagir pendant 2 heures à 90°C le TEMPO et le (1-bromoéthyl)benzène selon un rapport molaire TEMPO/(1-bromoéthyl)benzène égal à 2 (soit un excès molaire de TEMPO égal à 100 %), en présence d'un système organométallique [4,4'-di(5-nonyl)2,2'-bipyridine/ Cu $(OTf)_2$/Cu°].

On a maintenant trouvé un procédé de préparation d'alcoxyamines de formule

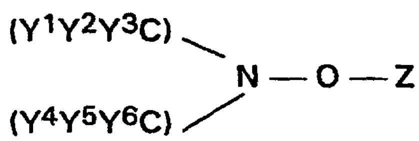

(I)

à partir de nitroxydes :

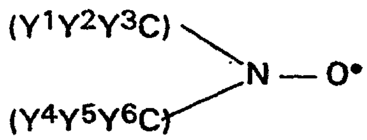

(II)

ledit procédé consistant à faire réagir ledit nitroxyde (II) avec un composé halogénocarboné ZX dans lequel X représente un atome de chlore, de brome ou d'iode, en milieu solvant organique non miscible à l'eau, en présence d'un système organométallique MA (L)n (III) dans lequel :

M représente un métal tel que Cu, Ag, Au,
A représente un atome d'halogène, un groupement carboxylate ou un groupement triflate,
L représente un ligand du métal M,
n vaut 1, 2 ou 3, selon le schéma :

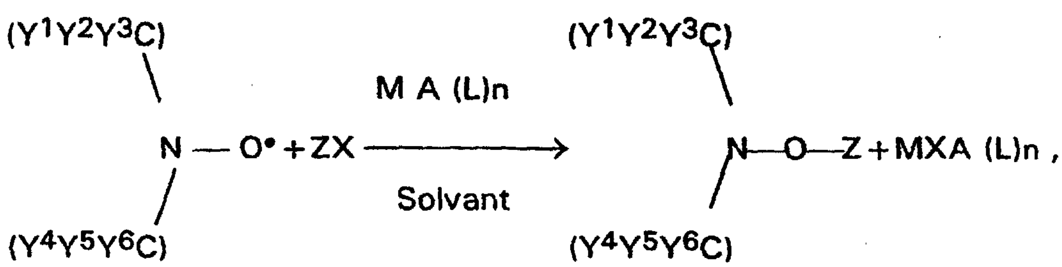


(II) (I)

ledit procédé étant caractérisé en ce qu'il consiste à effectuer les étapes suivantes :

a) on mélange sous agitation dans un solvant organique, un sel métallique MA, le ligand L, le composé halogéno-carboné ZX et le nitroxyde (II) selon un rapport molaire ZX / nitroxyde (II) allant de 1 à 1,4,
b) on maintient le milieu réactionnel sous agitation à une température comprise entre 20°C et 90°C jusqu'à disparition complète du nitroxyde (II),
c) on récupère la phase organique qui est lavée avec de l'eau, puis

d) on isole l'alcoxyamine (I) par évaporation du solvant organique sous pression réduite.

De préférence, M représente Cu; A représente un halogène tel que Cl ou Br, un groupement carboxylate tel qu'acétate ou un groupement triflate et X représente un atome de chlore ou un atome de brome.

Selon la présente invention, le ligand L du métal M du système organométallique (III) est choisi parmi les composés représentés par la formule générale (IV) :

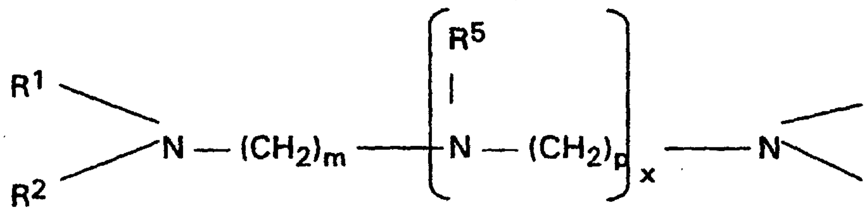

(IV)

dans laquelle $R^1$, $R^2$, $R^3$ et $R^4$, identiques ou différents, représentent un atome d'hydrogène, un groupe alkyle, linéaire ou ramifié ayant un nombre d'atomes de carbone allant de 1 à 10 et, de préférence allant de 1 à 4, $R^5$ représente un atome d'hydrogène, un groupe alkyle, linéaire ou ramifié, ayant un nombre d'atome de carbone allant de 1 à 10 et, de préférence, allant de 1 à 4, un reste

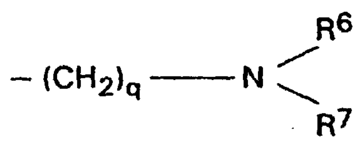

dans lequel $R^6$ et $R^7$ ont les mêmes significations que $R^5$, ou bien encore au moins deux des radicaux $R^1$, $R^2$, $R^3$, $R^4$ et $R^5$ peuvent être liés entre eux pour former un cycle ; m, p et q, identiques ou différents, représentent des nombres entiers allant de 1 à 4 et, de préférence, égaux à 2, x allant de 0 à 4.

A titre d'illustration de ligands L représentés par la formule (IV), on citera :

- la tris[2-(diméthylamino)éthyl]amine :

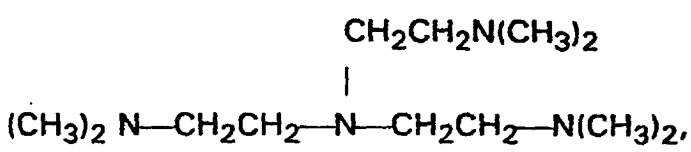

- la N, N, N', N', N" — pentaméthyldiéthylènetriamine (PMDETA) :

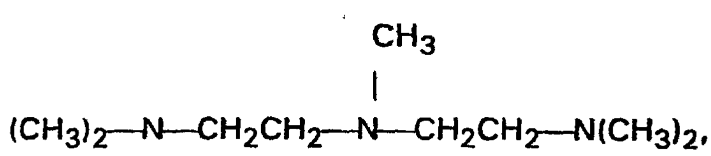

- la N,N,N',N'-tétraméthyléthylènediamine :

$(CH_3)_2—N—CH_2CH_2—N—CH_3)_2$,

- la 1, 1, 4, 7, 10, 10-hexaméthyltriéthylènetétramine (HMTETA) :

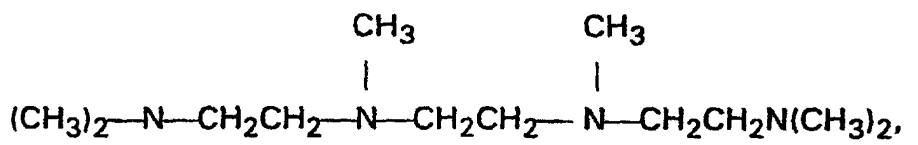

les polyamines cycliques telles que :

- le 1,4,7-triméthyl-1,4,7-triazacyclononane,
- le 1,5,9-triméthyl-1,5,9-triazacyclododécane,
- le 1,4,8,11-tétraméthyl-1,4,8,11-tétraazacyclotétradécane.

On utilisera de préférence la PMDETA.

Le procédé selon l'invention consiste à mélanger sous agitation un sel métallique MA, le ligand L, le composé ZX et le nitroxyde (II) dans un solvant organique qui est, de préférence, un hydrocarbure aromatique tel que le benzène, le toluène, les xylènes ou un chlorure d'alkyle tel que $CH_2Cl_2$ ou bien un éther.

Le degré d'oxydation de l'espèce active du métal M est égal à 1 ($M^I$).

Selon la présente invention, cette espèce active peut être ajoutée telle quelle au milieu réactionnel de préférence sous forme d'un halogénure métallique $M^IA$.

L'halogénure métallique préféré est CuBr.

L'espèce active peut être également générée in situ selon la réaction REDOX :

$$M^{II}A + M° \rightleftarrows 2M^IA$$

à partir d'un sel métallique $M^{II}A$ dans lequel le métal M est au degré d'oxydation 2 ($M^{II}$)et du même métal au degré d'oxydation zéro (M°).

Selon cette variante, l'halogénure métallique $M^{II}A$ préféré est $CuBr_2$.

Selon une autre variante, on peut également introduire dans le milieu réactionnel un sel métallique MA dans lequel le métal M est au degré d'oxydation 1 ($M^IA$) et le même métal M au degré d'oxydation zéro (M°).

Le ligand L est utilisé selon un rapport molaire $L/M^I$ allant de 1 à 5 et, de préférence, allant de 1 à 2.

Le rapport molaire ZX/nitroxyde (II) va de 1 à 1,4 et, de préférence est voisin de 1.

Le mélange réactionnel est agité ensuite à une température comprise entre 20°C et 90°C, et, de préférence, voisine de la température ambiante.

On opère sous atmosphère de gaz inerte tel que l'azote ou l'argon et de préférence à pression atmosphérique.

Les durées de réaction sont très courtes. La fin de réaction peut être contrôlée par disparition des réactifs par des méthodes chromatographiques (CPG, HPLC, CCM). La réaction terminée, le précipité éventuellement obtenu est filtré, rincé avec de préférence le même solvant utilisé pendant la réaction puis la phase organique est lavée avec de l'eau jusqu'à ce que les phases aqueuses extraites deviennent incolores.

Le solvant organique est éliminé sous pression réduite de préférence à température ambiante et on récupère l'alcoxyamine.

Selon l'invention, l'eau utilisée pour le lavage de la phase organique peut contenir un ou plusieurs sels selon des quantités pondérales au plus égales à la limite de solubilité desdits sels dans l'eau à température ambiante.

De préférence, on choisira ces sels parmi les sels des métaux alcalins, les sels d'ammonium ou les sels d'alkylammonium de chlorure, de formiate ou d'oxalate.

A titre d'illustration de tels sels utilisables selon la présente invention, on citera le chlorure de sodium, le formiate d'ammonium, le formiate de triéthylammonium, l'oxalate de diammonium.

Les alcoxyamines peuvent être caractérisées par analyse élémentaire, HPLC, IR et RMN.

Le procédé selon l'invention présente l'avantage d'être réalisé avec des ligands disponibles dans le commerce. La réaction entre le nitroxyde (II) et le composé halogénocarboné ZX est rapide. L'élimination du métal M du système organométallique MAa(L)n réalisée par de simples lavages à l'eau est particulièrement facile à mettre en oeuvre.

Le procédé selon l'invention permet d'obtenir des alcoxyamines exemptes quasiment de métal M.

Les alcoxyamines obtenues selon le procédé de l'invention, la teneur en métal M est inférieure à 10 ppm.

En outre, les rendements en alcoxyamine sont élevés.

Le procédé selon l'invention s'applique tout particulièrement à la préparation des alcoxyamines de formule

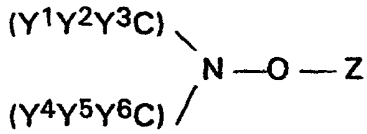

(I)

à partir de nitroxydes de formule :

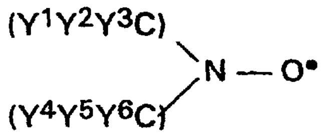

(II)

et de ZX

dans les formules desquelles les groupes $Y^1$ à $Y^6$, identiques ou différents, représentent un atome d'hydrogène, un radical alkyle, linéaire ou ramifié, ayant un nombre d'atomes de carbone allant de 1 à 10, un radical cycloalkyle ayant un nombre d'atomes de carbone allant de 3 à 20, un atome d'halogène, un radical cyano, un radical phényle, un radical hydroxyalkyle ayant un nombre d'atomes de carbone allant de 1 à 4, un radical dialcoxyphosphonyle, diphénoxyphosphonyle, un radical alcoxycarbonyle, alcoxycarbonylalkyle, ou bien 2 ou plus des groupes $Y^1$ à $Y^6$ peuvent être liés avec l'atome de carbone qui les porte pour former des structures cycliques, lesquelles peuvent comprendre une ou plusieurs fonctions extracycliques, choisis parmi : HO—, $CH_3C(O)$—, $CH_3O$—, $H_2N$—$CH_3C(O)NH$—, $(CH_3)_2N$— ; ou bien encore peuvent comprendre 1 ou plusieurs hétéroatomes extra- ou intracyliques tels que O, N;

Z est un reste de formule

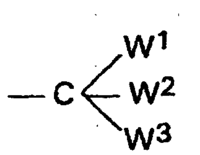

dans laquelle $W^1$, $W^2$ et $W^3$, identiques ou différents, représentent un atome d'hydrogène, un radical alkyle, linéaire ou ramifié, ayant un nombre d'atomes de carbone allant de 1 à 10, un radical phényle, un radical benzyle, un radical cyano, un radical cycloalkyle ayant un nombre d'atomes de carbone allant de 3 à 12 ; un radical $-(CH_2)r\ C(O)OW^4$ dans lequel $W^4$ représente un alkyle, linéaire ou ramifié, ayant un nombre d'atomes de carbone allant de 1 à 6 , r=0 à 6 ;

X représente un atome de chlore, de brome ou d'iode,

A titre d'illustration de nitroxydes (II) utilisables selon la présente invention, on citera :

- le 2,2,5,5 tétraméthyl-1-pyrrolidinyloxy (généralement commercialisé sous la marque PROXYL) ;
- le 3-carboxy-2,2,5,5-tétraméthyl-pyrrolidinyloxy (communément appelé 3-carboxy PROXYL) ;
- le 2,2,6,6-tétraméthyl-1-pipéridinyloxy (communément appelé TEMPO) ;
- le 4-hydroxy-2,2,6,6-tétraméthyl-1-pipéridinyloxy (communément appelé le 4-hydroxy-TEMPO) ;
- le 4-méthoxy-2,2,6, 6-tétraméthyl-1-pipéridinyloxy (communément appelé le 4-méthoxy-TEMPO) ;
- le 4-oxo-2,2,6,6-tétraméthyl-1-pipéridinyloxy (communément appelé le 4-oxo-TEMPO) ;
- le 4-amino-2,2,6,6-tétraméthyl-1-pipéridinyloxy (communément appelé le 4-amino-TEMPO) ;
- le 4-acétamido-2,2,6,6-tétraméthyl-1-pipéridinyloxy (communément appelé le 4-acétamido-TEMPO) ;
- le N-tertiobutyl-1-phényl-2-méthylpropyl nitroxyde,
- le N-(2-hydroxyméthylpropyl)-1-phényl-2-méthylpropylnitroxyde),
- le N-tertiobutyl-1-diéthylphosphono-2,2-diméthyl-propylnitroxyde,
- le N-tertiobutyl-1-dibenzylphosphono-2,2diméthylpropylnitroxyde,
- le N-tertiobutyl-1-di(2,2,2-trifluoroéthyl)phosphono-2,2diméthylpropylnitroxyde,
- le N-tertiobutyl-[(1-diéthylphosphono)-2-méthylpropyl]nitroxyde,

- le N-(1-méthyléthyl)-1-cyclohexyl-1-(diéthyl-phosphono)nitroxyde,
- le N-(1-phénylbenzyl)-[(1-diéthylphosphono)-1-méthyléthyl] nitroxyde :

  - le N-phényt-1-diéthylphosphono-2,2-diméthylpropytnitroxyde,
  - le N-phényl-1-diéthylphosphono-1-méthyléthyinitroxyde,
  - le N-(1-phényl2-méthylpropyl)-1-diéthylphosphonméthyléthylnitroxyde,
  - le bis-1-oxyl-2,2,6,6-tétraméthylpipéridine-4-yl)sébaçate commercialisé sous la marque "CXA 5415" par la Société CIBA SPEC. CHEM.

A titre d'illustration de composés ZX utilisables, on citera les composés de formule : $C_6H_5CH_2Br$, $(CH_3)_2C(CN)Br$, $CH_3OC(O)C(CH_3)_2Br$, $CH_3OC(O)CH(CH_3)Br$, $C_6F_{13}I$.

Les alcoxyamines de formule (I) obtenues selon le procédé de la présente invention peuvent être utilisées pour la polymérisation et la copolymérisation de tout monomère présentant une double liaison carbone-carbone susceptible de polymériser par voie radicalaire. La polymérisation ou la copolymérisation est réalisée dans les conditions habituelles connues de l'homme du métier compte tenu du ou des monomères considérés. Les monomères considérés peuvent être un monomère vinylaromatique (styrène, styrènes substitués), un diène, un monomère acrylique ou méthacrylique. Le monomère peut également être le chlorure de vinyle, le difluorure de vinylidène ou l'acrylonitrile.

Les exemples qui suivent illustrent l'invention.

## EXEMPLES

REMARQUES GENERALES

Les essais ont été réalisés sous atmosphère de gaz inerte (argon ou azote) en employant des techniques de Schlenk (standard)

Le 1-bromoéthylbenzène et le N-tertiobutyl-1-diéthylphosphono-2,2-diméthylpropylnitroxyde (DEPN) sont préalablement dégazés.

Les solvants utilisés sont le toluène qui est préalablement distillé sous argon sur sodium-benzophénone et $CH_2Cl_2$.

Les ligands utilisés sont :

- la N,N,N',N',N"-pentaméthyldiéthylènetriamine désignée ci-après par PMDETA,
- la tris(2-pyridylméthyl)amine désignée ci-après par TPA,
- la bipyridine désignée ci-après par BIPY.

Les alcoxyamines obtenues ont été caractérisées par RMN du $^1H$, $^{13}C$ et $^{31}P$, et par analyse élémentaire.

Les teneurs en cuivre résiduel ont été déterminées par la technique de spectroscopie d'émission atomique à plasma avec détection par spectrométrie de masse désignée ci-après ICP-MS (Inductively Coupled Plasma - Mass Spectrometry).

### EXEMPLE 1 (non conforme à l'invention)

PREPARATION DU N-TERTIOBUTYL, N-1-DIETHYLPHOSPHONO-2,2-DIMETHYLPROPYL, O-1-PHENYLETHYLHYDROXYLAMINE :

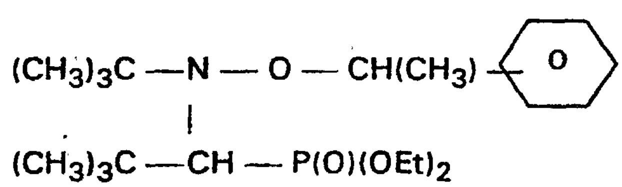

Dans un tube de Schlenk de 100 ml purgé à l'argon, on introduit 0,57 g de CuBr (4 mmol) et 1,25 g de BIPY (8 mmol) (rapport molaire BIPY/CuBr=2). On ajoute 0,74 g de (1-bromoéthyl)benzène (4 mmol) et 0,68 g de DEPN 86 % (2 mmol) dissous dans 9 ml de toluène anhydre. Sous agitation, on laisse réagir pendant 48 heures à température ambiante. Le mélange réactionnel est filtré sur célite. Le filtrat est lavé avec une solution aqueuse à 5 % de sulfate de cuivre, puis à l'eau. La phase organique est séchée sur sulfate de magnésium, puis le solvant est évaporé. On obtient une huile verdâtre contenant du cuivre qui est purifiée par chromatographie sur colonne de silice en utilisant un éluant pentane/éther 6/4. On obtient 0,75 g de N-tertiobutyl, N-1-diéthylphosphono-2,2-diméthylpropyl-O-1-phényléthylhydroxylamine (rendement = 95 %) sous la forme de deux diastéréoisomères dans des proportions 64/36 déterminées sur le spectre $^{31}P$ du mélange brut par intégration des signaux à 23,14 et 24,36 ppm (I/II = 64/36).

Les résultats analytiques sont donnés ci-après :

Isomère I :

RMN $^{31}P(CDCl_3)$ : $\delta$ 23,14
RMN $^{1}H(CDCl_3)$ : $\delta$ 0,88 (t,$J_{H-H}$=7,2Hz,3H) : 1,27 (m,21H) ; 1,55 (d, $J_{H-H}$=6,6Hz,3H) (s,9H) ; 3,40 (d,$J_{H-P}$=26Hz, 1H) ; 3,18-3,40 et 3,70-4,05 (m, 4H) ; 5,22 (q, $J_{H-H}$=6,6Hz, 1H) ; 7,24-7,47 (m,5H).
RMN $^{13}C$ $(CDCl_3)$ : $\delta$ 16,23 (2d,$J_{C-P}$=7Hz, $\underline{C}H_3CH_2$), 21,18 (s,$\underline{C}H_3CH$), 28,19 (s,$\underline{C}H_3$-C-CH), 30,63 (d,$J_{C-P}$=7Hz, $\underline{C}H_3$-CN); 35,33 (d,$J_{C-P}$=6Hz, $\underline{C}$-CH-P), 58,58 (d, $J_{C-P}$=7,5Hz, $\underline{C}$-$CH_3$), 61,4 (d, $J_{C-P}$=7Hz, $\underline{C}H_2$-O), 70,06 (d, $J_{C-P}$ = 138,5 Hz, $\underline{C}$H-P), 78,36 (s, $\underline{C}$H-O), 127,33 (s,$\underline{C}$H ar), 127,81 (s,$\underline{C}$H ar), 127,88 (s,$\underline{C}$H ar), 143,31 (s, $\underline{C}$ ar).
Microanalyse ($C_{21}H_{37}NO_4P$) : % calculé C 63,12 ; H 9,59 ; N 3,51. % trouvé C 63,01 ; H 9,60; N 3,42.

Isomère II :

RMN $^{31}P$ $(CDCl_3)$ : $\delta$ 24,36. RMN 1H $(CDCl_3)$ : $\delta$ 0,82 (s,9H) ; 1,22 (s,9H) ; 1,29 (t, $J_{H-H}$ 7,0Hz , 3H) ; 1,32 (t, $J_{H-H}$=7,0Hz, 3H) ; 1,58 (d, $J_{H-H}$=6,7Hz, 3H) ; 3,32 (d, $J_{H-P}$=26,2Hz, 1H) ; 3,9-4,2 et 4,3-4,4 (m, 4H) ; 4,97 (q, $J_{H-H}$=6,8Hz, 1H) ; 7,17-7,3 (m, 5H).
RMN $^{13}C$ $(CDCl_3)$ : $\delta$ 16,24 (d,$J_{C-P}$=7,1Hz, $\underline{C}H_3CH_2$), 16,71 (d, $J_{C-P}$=5,2Hz, $\underline{C}H_3CH_2$), 24,00 (s, $\underline{C}H_3CH$), 28,50 (s, $\underline{C}H_3$-C-CH), 30,12 (d, $J_{C-P}$=5,7Hz, $\underline{C}H_3$-C-N), 35,37 (d, $J_{C-P}$=5,8Hz, $\underline{C}$-$C_{H-P}$), 58,80 (d, $J_{C-P}$=7,4Hz, $\underline{C}H_2$-O), 61,10 (s, C-N), 61,56 (d, $J_{C-P}$=6Hz, $\underline{C}H_2$-O), 69,84 (d, $J_{C-P}$=138,4Hz, $\underline{C}$H-P), 85,23 (s, $\underline{C}$H-O), 126,96 (s, $\underline{C}$H ar), 127,08 (s, $\underline{C}$H ar), 127,95 (s, $\underline{C}$H ar), 145,36 (s, $\underline{C}$ ar).
Microanalyse ($C_{21}H_{37}NO_4P$) : % calculé C 63,12 ; H 9,59 ; N 3,51. % trouvé C 63,05 ; H 9,51 ; N 3,50.

**EXEMPLE 2 (conforme à l'invention)**

PREPARATION DU N-TERTIOBUTYL, N-1-DIETHYLPHOSPHONO-2,2-DIMETHYLPROPYL, O-1-PHENYLETHYLHYDROXYLAMINE :

Utilisation de la PMDETA au lieu de la BIPY:

Dans un tube de Schlenk de 100 ml, on introduit 0,46 g de CuBr (3,21 mmol) et 1,11 g de PMDETA (6,42 mmol). On purge par des séquences vide-argon, puis on ajoute 0,59 g de (1-bromoéthyl)benzène (3,21 mmol) et 1 g de DEPN 70 % (2,38 mmol) dilué dans 10 ml de toluène. Sous agitation, on laisse réagir pendant 30 minutes à température ambiante. Le mélange réactionnel est filtré sur célite, puis le filtrat est lavé à l'eau (5 fois 30 ml d'eau). Le solvant est évaporé pour donner 0,98 g de N-tertiobutyl, N-1-diéthylphosphono-2,2-diméthylpropyl, O-1-phényléthylhydroxylamine qui se présente sous forme d'une huile incolore.

La pureté du produit analysé par HPLC est de 97 %. Le rendement est voisin de 100 %. Les caractéristiques analytiques sont identiques à l'alcoxyamine obtenue dans l'exemple 1.

La teneur en Cu résiduel est inférieure à 10 ppm.

**EXEMPLE 3 (conforme à l'invention)**

PREPARATION DU 1-(2,2,6,6-TETRAMETHYLPIPERIDINYLOXY)-1-PHENYLETHANE :

La réaction se déroule sous atmosphère d'azote.

Dans un tube de Schlenk de 100 ml, on introduit 10 ml de toluène, 0,4 g de cuivre, 0,84 g de CuBr et 1 g de PMDETA.

La solution est dégazée sous pression réduite puis on y ajoute 10 ml de toluène dégazé contenant 0,92 g de

TEMPO (0,0059 mol) et 1,1 g de (1-bromoéthyl)benzène (0,0059 mole).

L'addition est exothermique. Au bout de 30 minutes, on vérifie par chromatographie sur couches minces (CCM) que les réactifs ont disparu. On filtre la solution organique puis on lave avec de l'eau jusqu'à ce que les phases aqueuses soient incolores. On évapore ensuite le toluène et on obtient 1,5 g de 1-(2,2,6,6-tétraméthylpipéridinyloxy)-1-phényléthane (rendement 97 %) dont la pureté est vérifiée par RMN du $^1H$ et $^{13}C$ par comparaison avec les données de la littérature.

Le dosage du cuivre par ICP-MS montre que la teneur en cuivre est inférieure à 10 ppm.

**EXEMPLES 4(NC), 5(NC) et 6 (conforme à l'invention)**

Nous avons réalisé la préparation du N-tertiobutyl, N-1-diéthylphosphono-2,2-diméthylpropyl, 0-1-phényléthylhydroxylamine selon un mode opératoire similaire à celui de l'exemple 2 avec différents ligands L et selon des conditions opératoires rapportées dans le tableau 1. Les résultats obtenus sont reportés dans ce tableau 1. Les exemples 4 (NC), et 5 (NC) sont non conformes à l'invention.

**EXEMPLE 7 (conforme à l'invention)**

On reconduit la réaction entre le DEPN et le (1-bromoéthyl)benzène dans les mêmes conditions que celles décrites dans l'exemple 2 excepté que l'on remplace le toluène par $CH_2Cl_2$. L'analyse par CCM après 5 minutes de réaction montre que tous les réactifs ont réagi.

Après lavages à l'eau, on obtient le N-tertiobutyl, N-1-diéthylphosphono-2,2-diméthylpropyl, 0-1-phényléthylhydroxylamine avec un rendement de 91 %. La teneur en cuivre est inférieure à 10 ppm. Les résultats de cet exemple sont reportés également dans le tableau 1.

TABLEAU 1

| Exemple | L | Solvant | $\frac{L}{Cu^T}$ | $\frac{ZX}{DEPN}$ | Temps (min) | Rendement | Cu (ppm) |
|---|---|---|---|---|---|---|---|
| 4 (NC) | BIPY | toluène | 2 | 2 | 240 | 2 | - |
| 5 (NC) | TPA | toluène | 2 | 1,35 | 240 | 85 | 100 |
| 6 | PMDETA | toluène | 2 | 1 | 60 | 95 | <10 |
| 7 | PMDETA | $CH_2Cl_2$ | 1 | 1 | 5 | 91 | <10 |

**EXEMPLE 8 (conforme à l'invention)**

PREPARATION DU N-TERTIOBUTYL, N-1-DIETHYLPHOSPHONO-2,2-DIMETHYLPROPYL, 0,1-PHENYLETHYLHYDROXYLAMINE :

Dans cet exemple, le traitement est effectué avec une solution de formiate de triéthylammonium. Le formiate de triéthylammonium est préparé en mélangeant de l'acide formique et de la triéthylamine dans des proportions molaires 1,5/1.

Dans un réacteur de 250 ml purgé à l'argon, on charge 4,3 g de (1-bromoéthyl)benzène (0,023 mol), 5,4 g de DEPN 93 % (0,017 mol), 3,3 g de CuBr (0,023 mol), 4,0 g de PMDETA (0,023 mol), 1,45 de poudre de cuivre (0,023 mol) et 50 g de toluène dégazé. On laisse réagir 3h sous agitation à 35°C. Le mélange réactionnel est filtré sur Célite. Le filtrat est lavé avec 25 g d'une solution aqueuse à 40 % poids de formiate de triéthylammonium, puis avec de l'eau (2 x 25 g). La phase organique est évaporée sous vide pour donner 6,1 g de N-tertiobutyl, N-1-diéthylphosphono-2,2-diméthylpropyl-0-1-phényléthylhydroxylamine sous forme d'une huile incolore (rendement = 90 % ; pureté = 97 %). La teneur en Cu résiduel est inférieure à 10 ppm.

**EXEMPLE 9**

PREPARATION DU N-TERTIOBUTYL, N-1-DIETHYLPHOSPHONO2,2-DIMETHYLPROPYL, 0,1-METHOXYCARBONYLETHYLHYDROXYLAMINE :

Dans un réacteur de 2 l purgé à l'argon, on charge 115 g de 2-bromopropionate de méthyle (0,687 mol), 200 g de DEPN 91 % (0,619 mol), 49,3 g de CuBr (0.344 mol), 59,8 g de PMDETA (0,344 mol), 43,6 g de poudre de cuivre

(0,687 mol) et 800 ml de toluène dégazé. On laisse réagir 4 h sous agitation à température ambiante. Le mélange réactionnel est filtré sur Célite. Le filtrat est lavé avec une solution aqueuse à 40 % poids de formiate d'ammonium (2 x 500 ml), puis avec une solution aqueuse à 5 % d'hydrogénocarbonate de potassium (1 x 500 ml). La phase organique est évaporée sous vide pour donner 212 g de N-tertiobutyl, N-1-diéthylphosphono-3,2-diméthylpropyl, 0-1-méthoxycarbonyléthylhydroxylamine sous forme d'huile légèrement jaune (rendement = 90 %, pureté = 98 %). La teneur en Cu résiduel est inférieure à 10 ppm.

## Revendications

**1.** Procédé de préparation d'alcoxyamines de formule :

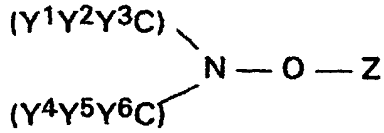

(I)

à partir de nitroxydes de formule :

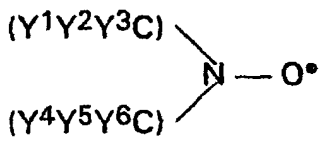

(II)

et de ZX

dans les formules desquelles les groupes $Y^1$ à $Y^6$, identiques ou différents, représentent un atome d'hydrogène, un radical alkyle, linéaire ou ramifié, ayant un nombre d'atomes de carbone allant de 1 à 10, un radical cycloalkyle ayant un nombre d'atomes de carbone allant de 3 à 20, un atome d'halogène, un radical cyano, un radical phényle, un radical hydroxyalkyle ayant un nombre d'atomes de carbone allant de 1 à 4, un radical dialcoxyphosphonyle, diphénoxyphosphonyle, un radical alcoxycarbonyle, alcoxycarbonylalkyle, ou bien 2 ou plus des groupes $Y^1$ à $Y^6$ peuvent être liés avec l'atome de carbone qui les porte pour former des structures cycliques, lesquelles peuvent comprendre une ou plusieurs fonctions extracycliques, choisis parmi : HO—, $CH_3C(O)$—, $CH_3O$—, $H_2N$—$CH_3C(O)NH$—, $(CH_3)_2N$— ; ou bien encore peuvent comprendre 1 ou plusieurs hétéroatomes extra- ou intracyliques tels que O, N ;

Z est un reste de formule

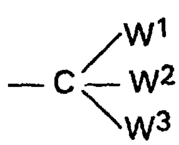

dans laquelle $W^1$, $W^2$ et $W^3$, identiques ou différents, représentent un atome d'hydrogène, un radical alkyle, linéaire ou ramifié, ayant un nombre d'atomes de carbone allant de 1 à 10, un radical phényle, un radical benzyle, un radical cyano, un radical cycloalkyle ayant un nombre d'atomes de carbone allant de 3 à 12 ; un radical $-(CH_2)rC(O)OW^4$ dans lequel $W^4$ représente un alkyle, linéaire ou ramifié, ayant un nombre d'atomes de carbone allant de 1 à 6 , r=0 à 6 ;

X représente un atome de chlore, de brome ou d'iode,

ledit procédé consistant à faire réagir ledit nitroxyde (II) avec un composé halogénocarboné ZX, en milieu solvant organique non miscible à l'eau, en présence d'un système organométallique MA (L)n (III) dans lequel:

M représente un métal tel que Cu, Ag, Au,
A représente un atome d'halogène, un groupement carboxylate ou un groupement triflate,
L représente un ligand du métal M,
n vaut 1, 2 ou 3, selon le schéma

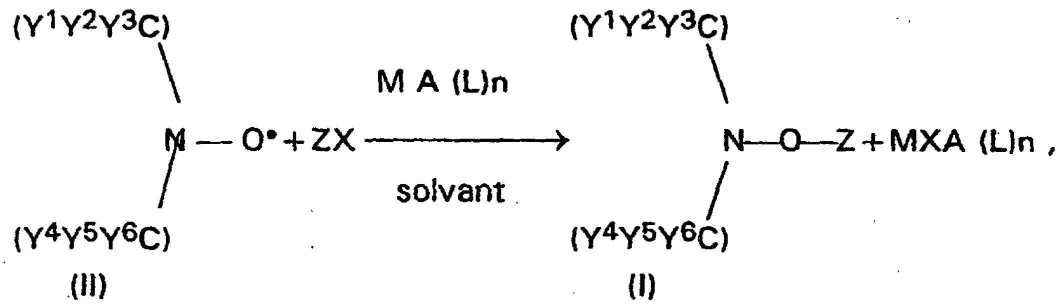


ledit procédé étant **caractérisé en ce qu'**il consiste à effectuer les étapes suivantes:

a) on mélange sous agitation dans un solvant organique, un sel métallique MA, le ligand L, le composé halogéno-carboné ZX et le nitroxyde (II) selon un rapport molaire ZX / nitroxyde (II) allant de 1 à 1,4,
b) on maintient le milieu réactionnel sous agitation à une température comprise entre 20°C et 90°C jusqu'à disparition complète du nitroxyde (II),
c) on récupère la phase organique qui est lavée avec de l'eau, puis
d) on isole l'alcoxyamine (I) par évaporation du solvant organique sous pression réduite.

2. Procédé selon la revendication 1, **caractérisé en ce que** le ligand L du métal M du système organométallique (III) est choisi parmi les composés représentés par la formule générale (IV):

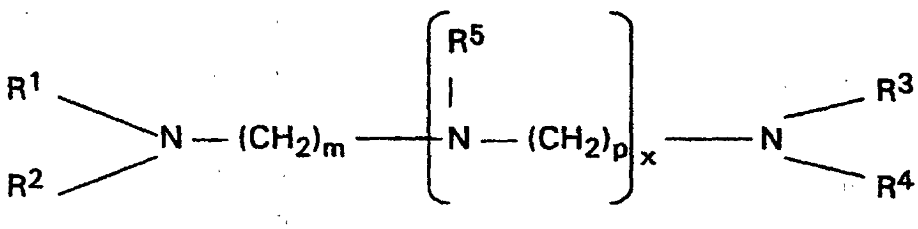

(IV)

dans laquelle $R^1$, $R^2$, $R^3$ et $R^4$, identiques ou différents, représentent un atome d'hydrogène, un groupe alkyle, linéaire ou ramifié ayant un nombre d'atomes de carbone allant de 1 à 10 et, de préférence allant de 1 à 4, $R^5$ représente un atome d'hydrogène, un groupe alkyle, linéaire ou ramifié, ayant un nombre d'atome de carbone allant de 1 à 10 et, de préférence, allant de 1 à 4, un reste

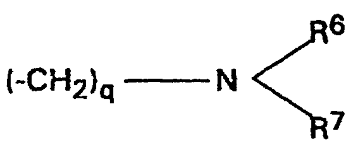

dans lequel $R^6$ et $R^7$ ont les mêmes significations que $R^5$, ou bien encore au moins deux des radicaux $R^1$, $R^2$, $R^3$, $R^4$ et $R^5$ peuvent être liés entre eux pour former un cycle ; m, p et q, identiques ou différents, représentent des nombres entiers allant de 1 à 4 et, de préférence, égaux à 2, x allant de 0 à 4.

3. Procédé selon la revendication 1 ou 2, **caractérisé par le fait que** M représente Cu.

4. Procédé selon l'une des revendications 1 à 3, **caractérisé par le fait que** A représente un atome de brome et X représente un atome de chlore ou un atome de brome.

5. Procédé selon l'une des revendications 1à 4 **caractérisé en ce que** le rapport molaire ZX / nitroxyde (II) est voisin de 1.

6. Procédé selon l'une des revendications 1 à 4, **caractérisé en ce que** le sel métallique MA est un halogénure métallique $M^IA$.

7. Procédé selon la revendication 6, **caractérisé en ce que** l'halogénure métallique $M^IA$ est CuBr.

8. Procédé selon l'une des revendications 1à 7, **caractérisé en ce que** le rapport molaire L / $M^I$ va de 1 à 5.

9. Procédé selon la revendication 8, **caractérisé en ce que** le rapport molaire L / $M^I$ va de 1 à 2.

10. Procédé selon la revendication 1, **caractérisé en ce que** le solvant organique est un hydrocarbure aromatique ou un chlorure d'alkyle.

11. Procédé selon la revendication 10, **caractérisé en ce que** l'hydrocarbure aromatique est le toluène et le chlorure d'alkyle est le chlorure de méthylène.

12. Procédé selon l'une des revendications 1 à 11, **caractérisé en ce que** le ligand L est :

- la tris[2-(diméthylamino)éthyl]amine :

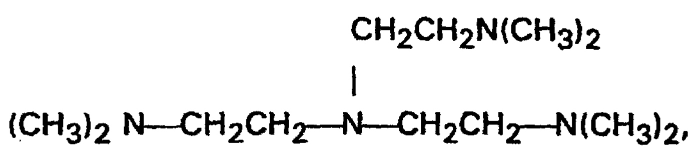

- la N, N, N', N', N" — pentaméthyldiéthylènetriamine (PMDETA) :

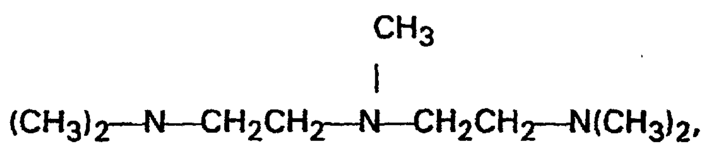

- la N,N,N',N'-tétraméthyléthylènediamine :

$$(CH_3)_2—N—CH_2CH_2—N—CH_3)_2,$$

- la 1, 1, 4, 7, 10, 10-hexaméthyltriéthylènetétramine (HMTETA) :

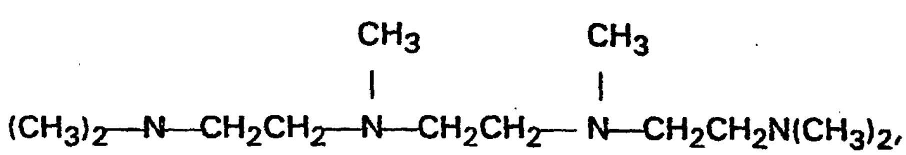

les polyamines cycliques telles que:
- le 1,4,7-triméthyl-1,4,7-triazacyclononane,
- le 1,5,9-triméthyl-1,5,9-triazacyclododécane,
- le 1,4,8,11-tétraméthyl-1,4,8,11-tétraazacyclotétradécane.

13. Procédé selon la revendication 12, **caractérisé en ce que** le ligand L est la N,N,N',N',N"-pentaméthyldiéthylénetriamine (PMDETA).

**14.** Procédé selon la revendication 1, **caractérisé en ce que** l'eau utilisée pour le lavage de la phase organique contient un ou plusieurs sels choisis parmi les sels de métaux alcalins, les sels d'ammonium ou les sels d'alkylammonium de chlorure, de formiate ou d'oxalate.

**15.** Procédé selon la revendication 14, **caractérisé en ce que** le sel est le formiate de triéthylammonium.

**16.** Procédé selon la revendication 14, **caractérisé en ce que** le sel est le formiate d'ammonium.

## Claims

**1.** Process for preparing alkoxyamines of formula:

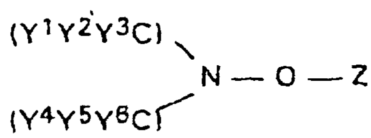

(I)

from nitroxides of formula:

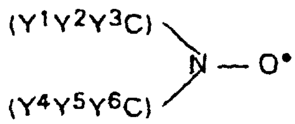

(II)

and ZX

in which formulae the groups $Y^1$ to $Y^6$, which may be identical or different, represent a hydrogen atom, a linear or branched alkyl radical containing a number of carbon atoms ranging from 1 to 10, a cycloalkyl radical containing a number of carbon atoms ranging from 3 to 20, a halogen atom, a cyano radical, a phenyl radical, a hydroxyalkyl radical containing a number of carbon atoms ranging from 1 to 4, a dialkoxyphosphonyl or diphenoxyphosphonyl radical, an alkoxycarbonyl or alkoxycarbonylalkyl radical, or alternatively two or more of the groups $Y^1$ to $Y^6$ can be linked with the carbon atom which bears them to form cyclic structures, which can comprise one or more exocyclic functions chosen from: HO-, $CH_3C(O)$-, $CH_3O$-, $H_2N$-$CH_3C(O)NH$-, $(CH_3)_2N$-; or alternatively can comprise one or more exocyclic or endocyclic hetero atoms such as O or N;

Z is a residue of formula

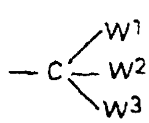

in which $W^1$, $W^2$ and $W^3$, which may be identical or different, represent a hydrogen atom, a linear or branched alkyl radical containing a number of carbon atoms ranging from 1 to 10, a phenyl radical, a benzyl radical, a cyano radical, a cycloalkyl radical containing a number of carbon atoms ranging from 3 to 12; a radical $-(CH_2)rC(O)OW^4$ in which $W^4$ represents a linear or branched alkyl containing a number of carbon atoms ranging from 1 to 6, r = 0 to 6;

X represents a chlorine, bromine or iodine atom,

the said process consisting in reacting the said nitroxide (II) with a halocarbon compound ZX, in a water-immiscible organic solvent medium, in the presence of an organometallic system MA (L)n (III) in which:

M represents a metal such as Cu, Ag or Au,
A represents a halogen atom, a carboxylate group or a triflate group,
Lrepresents a ligand for the metal M,
n is 1, 2 or 3, according to the scheme:

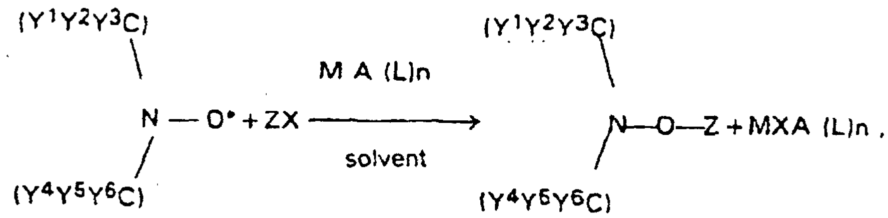


(II) (I)

the said process being **characterized in that** it consists in carrying out the following steps:

a) a metal salt MA, the ligand L, the halocarbon compound ZX and the nitroxide (II) are mixed together with stirring, in an organic solvent, in a ZX/nitroxide (II) molar ratio ranging from 1 to 1.4,
b) the reaction medium is kept stirring at a temperature of between 20°C and 90°C until the nitroxide (II) has completely disappeared,
c) the organic phase is recovered and washed with water, and then
d) the alkoxyamine (I) is isolated by evaporating the organic solvent under reduced pressure.

**2.** Process according to Claim 1, **characterized in that** the ligand L for the metal M in the organometallic system (III) is chosen from the compounds represented by the general formula (IV):

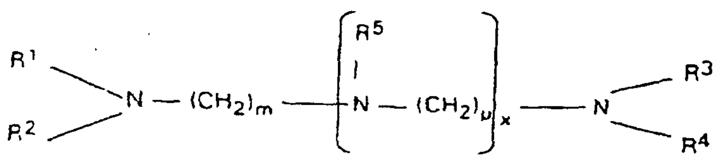

(IV)

in which $R^1$, $R^2$, $R^3$ and $R^4$, which may be identical or different, represent a hydrogen atom, a linear or branched alkyl group containing a number of carbon atoms ranging from 1 to 10 and preferably ranging from 1 to 4, $R^5$ represents a hydrogen atom, a linear or branched alkyl group containing a number of carbon atoms ranging from 1 to 10 and preferably ranging from 1 to 4, a residue

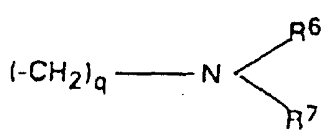

in which $R^6$ and $R^7$ have the same meanings as $R^5$, or alternatively at least two of the radicals $R^1$, $R^2$, $R^3$, $R^4$ and $R^5$ may be linked together to form a ring; m, p and q, which may be identical or different, represent integers ranging from 1 to 4 and preferably equal to 2, x ranging from 0 to 4.

**3.** Process according to Claim 1 or 2, **characterized in that** M represents Cu.

**4.** Process according to one of Claims 1 to 3, **characterized in that** A represents a bromine atom and X represents a chlorine atom or a bromine atom.

**5.** Process according to one of Claims 1 to 4, **characterized in that** the ZX/nitroxide (II) molar ratio is in the region of 1.

**6.** Process according to one of Claims 1 to 4, **characterized in that** the metal salt MA is a metal halide $M^IA$.

**7.** Process according to Claim 6, **characterized in that** the metal halide $M^IA$ is CuBr.

**8.** Process according to one of Claims 1 to 7, **characterized in that** the molar ratio $L/M^I$ ranges from 1 to 5.

**9.** Process according to Claim 8, **characterized in that** the molar ratio $L/M^I$ ranges from 1 to 2.

**10.** Process according to Claim 1, **characterized in that** the organic solvent is an aromatic hydrocarbon or an alkyl chloride.

**11.** Process according to Claim 10, **characterized in that** the aromatic hydrocarbon is toluene and the alkyl chloride is methylene chloride.

**12.** Process according to one of Claims 1 to 11, **characterized in that** the ligand L is:

- tris[2-(dimethylamino)ethyl]amine:

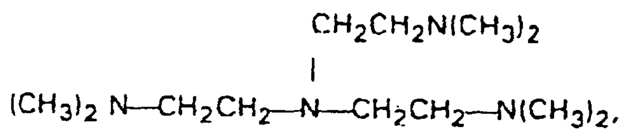

- N,N,N',N',N"-pentamethyldiethylenetriamine (PMDETA):

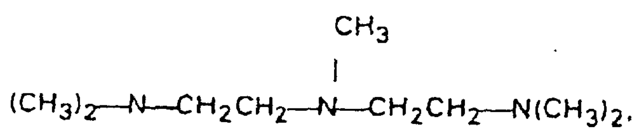

- N,N,N',N'-tetramethylethylenediamine:

$$(CH_3)_2\text{-N-}CH_2CH_2\text{-N-}CH_3)_2,$$

- 1,1,4,7,10,10-hexamethyltriethylenetetramine (HMTETA):

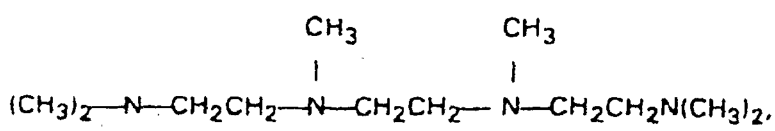

cyclic polyamines such as:

- 1,4,7-trimethyl-1,4,7-triazacyclononane,
- 1,5,9-trimethyl-1,5,9-triazacyclododecane,
- 1,4,8,11-tetramethyl-1,4,8,11-tetraazacyclotetradecane.

**13.** Process according to Claim 12, **characterized in that** the ligand L is N,N,N',N',N"-pentamethyldiethylenetriamine (PMDETA).

**14.** Process according to Claim 1, **characterized in that** the water used to wash the organic phase contains one or more salts chosen from alkali metal salts, ammonium salts and alkylammonium salts of chloride, formate or oxalate.

**15.** Process according to Claim 14, **characterized in that** the salt is triethylammonium formate.

**16.** Process according to Claim 14, **characterized in that** the salt is ammonium formate.

**Patentansprüche**

**1.** Verfahren zur Herstellung von Alkoxyaminen der Formel

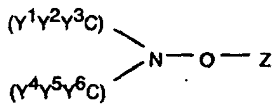

(I)

ausgehend von Nitroxiden der Formel

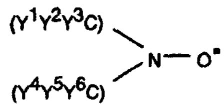

(II)

und ZX

wobei in den Formeln

die Gruppen $Y^1$ bis $Y^6$, identisch oder verschieden, ein Wasserstoffatom, einen linearen oder verzweigten Alkylrest mit 1 bis 10 Kohlenstoffatomen, einen Cycloalkylrest mit 3 bis 20 Kohlenstoffatomen, ein Halogenatom, einen Cyanorest, einen Phenylrest, einen Hydroxyalkylrest mit 1 bis 4 Kohlenstoffatomen, einen Dialkoxyphosphonylrest, einen Diphenoxyphosphonylrest, einen Alkoxycarbonylrest oder einen Alkoxycarbonylalkylrest darstellen oder aber 2 oder mehrere der Gruppen $Y^1$ bis $Y^6$ mit einem Kohlenstoffatom, das sie trägt, verbunden sein können, um ringförmige Strukturen zu bilden, die ein oder mehrere Funktionen außerhalb des Rings, ausgewählt aus HO-, $CH_3C(O)$-, $CH_3O$-, $H_2N$-$CH_3C(O)NH$- und $(CH_3)_2N$-, umfassen können oder aber 1 oder mehrere Heteroatome, wie O oder N, außerhalb oder innerhalb des Rings umfassen können;

Z einen Rest der Formel

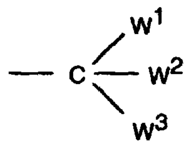

ist, in der $W^1$, $W^2$, und $W^3$, identisch oder verschieden, ein Wasserstoffatom, einen linearen oder verzweigten Alkylrest mit 1 bis 10 Kohlenstoffatomen, einen Phenylrest, einen Benzylrest, einen Cyanorest, einen Cycloalkylrest mit 3 bis 12 Kohlenstoffatomen

oder einen Rest $-(CH_2)_rC(O)OW^4$, wobei $W^4$ einen linearen oder verzweigten Alkylrest mit 1 bis 6 Kohlenstoffatomen darstellt und

r = 0 bis 6 ist, darstellen;

X ein Chlor-, Brom- oder Iodatom darstellt,

wobei das Verfahren darin besteht, das Nitroxid (II) mit einer Halogenkohlenstoffverbindung ZX in einem nicht mit Wasser mischbaren organischen Lösemittelmilieu in Gegenwart eines Organometallsystems $MA(L)_n$(III),

wobei in der Formel (III)

M ein Metall wie Cu, Ag oder Au darstellt,

A ein Halogenatom, eine Carboxylatgruppe oder eine Triflatgruppe darstellt,

L einen Liganden des Metalls M darstellt und

n 1,2 oder 3 ist,

gemäß dem Reaktionsschema

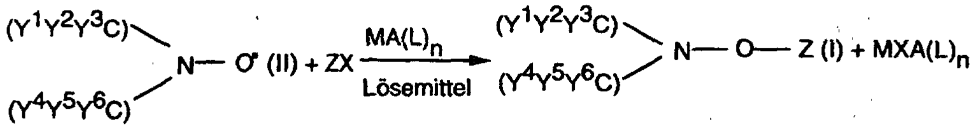


reagieren zu lassen,
**dadurch gekennzeichnet,**
**daß** man die folgenden Verfahrensschritte durchführt:

a) man mischt unter Rühren in einem organischen Lösemittel ein Metallsalz MA, den Liganden L, die Halogenenkohlenstoffverbindung ZX und das Nitroxid (II) in einem ZX/Nitroxid (II)-Molverhältnis von 1 bis 1,4,

b) man beläßt das Reaktionsmilieu unter Rühren bei einer Temperatur zwischen 20 °C und 90 °C bis zum vollständigen Verschwinden des Nitroxids (II),

c) man erhält die organische Phase, die mit Wasser gewaschen wird, dann

d) isoliert man das Alkoxyamin (I) durch Abdampfen des organischen Lösemittels unter reduziertem Druck.

**2.** Verfahren nach Anspruch 1, **dadurch gekennzeichnet, daß** der Ligand des Metalls M des Organometallsystems (III) ausgewählt ist aus Verbindungen der allgemeinen Formel (IV)

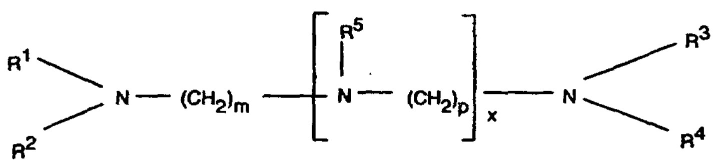

(IV)

in der $R^1$, $R^2$, $R^3$ und $R^4$, identisch oder verschieden, ein Wasserstoffatom oder eine lineare oder verzweigte Alkylgruppe mit 1 bis 10 Kohlenstoffatomen, vorzugsweise 1 bis 4 Kohlenstoffatomen, darstellen, $R^5$ ein Wasserstoffatom, einen linearen oder verzweigten Alkylrest mit 1 bis 10 Kohlenstoffatomen, vorzugsweise 1 bis 4 Kohlenstoffatomen, oder einen Rest

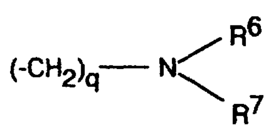

darstellen, in dem $R^6$ und $R^7$ dieselben Bedeutungen wie $R^5$ hat, oder aber mindestens zwei der Reste $R^1$, $R^2$, $R^3$, $R^4$ und $R^5$ untereinander verbunden sein können, um einen Ring zu bilden; m, p und q, identisch oder verschieden, eine ganze Zahl von 1 bis 4, vorzugsweise gleich 2, darstellen und x von 0 bis 4 reicht.

**3.** Verfahren nach Anspruch 1 oder 2, **dadurch gekennzeichnet, daß** M Cu darstellt.

**4.** Verfahren nach einem der Ansprüche 1 bis 3, **dadurch gekennzeichnet, daß** A ein Bromatom darstellt und X ein Chlor- oder Bromatom darstellt.

**5.** Verfahren nach einem der Ansprüche 1 bis 4, **dadurch gekennzeichnet, daß** das ZX/Nitroxid (II)-Molverhältnis etwa 1 beträgt.

**6.** Verfahren nach einem der Ansprüche 1 bis 4, **dadurch gekennzeichnet, daß** das Metallsalz MA ein Metallhalogenid M'A ist.

**7.** Verfahren nach Anspruch 6, **dadurch gekennzeichnet, daß** das Metallhalogenid M'A CuBr ist.

8. Verfahren nach einem der Ansprüche 1 bis 7, **dadurch gekennzeichnet, daß** das L/M'-Molverhältnis von 1 bis 5 reicht.

9. Verfahren nach Anspruch 8, **dadurch gekennzeichnet, daß** das L/M'-Molverhältnis von 1 bis 2 reicht.

10. Verfahren nach Anspruch 1, **dadurch gekennzeichnet, daß** das organische Lösemittel ein aromatischer Kohlenwasserstoff oder ein Alkylchlorid ist.

11. Verfahren nach Anspruch 10, **dadurch gekennzeichnet, daß** der aromatische Kohlenwasserstoff Toluol ist und daß das Alkylchlorid Methylenchlorid ist.

12. Verfahren nach einem der Ansprüche 1 bis 11, **dadurch gekennzeichnet, daß** der Ligand L eine der folgenden Gruppen ist:

- Tris-[2-(dimethylamino)ethyl]amin

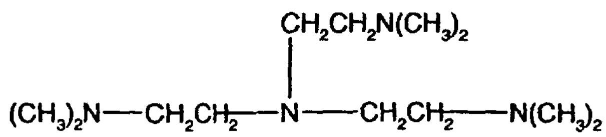

- N,N,N',N',N''-Pentamethyldiethylentriamin (PMDETA)

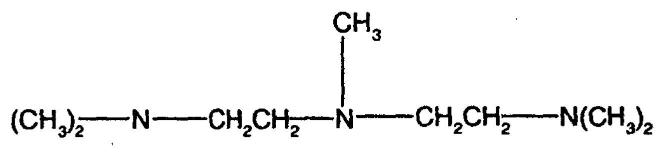

- N,N,N',N'-Tetramethylethylendiamin

$$(CH_3)_2—N—CH_2CH_2—N—(CH_3)_2$$

- 1,1,4,7,10,10-Hexamethyltriethylentetramin (HMTETA)

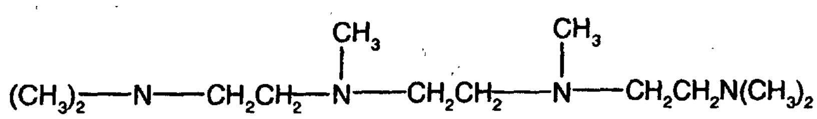

- ringförmige Polyamine wie
  - 1,4,7-Trimethyl-1,4,7-triazacyclononan
  - 1,5,9-Trimethyl-1,5,9-triazacyclododekan
  - 1,4,8,11-Tetramethyl-1,4,8,11-tetraazacyclotetradekan.

13. Verfahren nach Anspruch 12, **dadurch gekennzeichnet, daß** der Ligand L N,N,N',N',N''-Pentamethyldiethylentriamin (PMDETA) ist.

14. Verfahren nach Anspruch 1, **dadurch gekennzeichnet, daß** das für das Waschen der organischen Phase verwendete Wasser ein oder mehrere Salze enthält, die ausgewählt sind aus Alkalimetallsalzen, Ammoniumsalzen

oder Alkylammoniumchlorid-, -formiat- oder -oxalatsalzen.

**15.** Verfahren nach Anspruch 14, **dadurch gekennzeichnet, daß** das Salz Triethylammoniumformiat ist.

**16.** Verfahren nach Anspruch 14, **dadurch gekennzeichnet, daß** das Salz Ammoniumformiat ist.